# EUROPÄISCHE PATENTSCHRIFT

(11) **EP 3 038 744 B1**
(45) Veröffentlichungstag und Bekanntmachung des Hinweises auf die Patenterteilung: **20.12.2017**
(21) Anmeldenummer: 14758343.9
(22) Anmeldetag: 27.08.2014
(51) Int. Cl.: B01J 20/24, B01J 20/28, C09K 3/32, C02F 1/28, C02F 1/68, C02F 101/00, C02F 101/32, C02F 103/00, C02F 3/34, E02B 15/10, C12N 11/02, D04H 1/00, D04H 1/425, D04H 1/732

(54) **PORÖSER ÖLBINDER UND DESSEN VERWENDUNG ZUM ABBAU UND/ODER ZUR ENTFERNUNG VON ÖLVERSCMUTZUNGEN**
POROUS OIL BINDER AND USE THEREOF FOR DECOMPOSITION AND/OR REMOVAL OF OIL CONTAMINATIONS
ADSORBANT D'HUILLE POREUX ET UTILISATION DUDIT ADSORBANT DANS LA DÉCOMPOSITION ET/OU L'EXTRACTION DES CONTAMINATIONS HUILEUSES

(30) Priorität: 27.08.2013 DE 102013217016
(43) Veröffentlichungstag der Anmeldung: 06.07.2016
(73) Patentinhaber: Technische Universität Dresden, 01069 Dresden (DE); Universität Leipzig, 04109 Leipzig (DE)
(72) Erfinder: UNBEHAUN, Holger, 01097 Dresden (DE); TECH, Sören, 01277 Dresden (DE); KÖNIG, Swetlana, 04229 Leipzig (DE); SAFONOVA, Elena, 04299 Leipzig (DE); WAGENFÜHR, André, 01109 Dresden (DE); WILHELM, Christian, 04416 Markleeberg (DE)
(74) Vertreter: Kailuweit & Uhlemann Patentanwälte Partnerschaft mbB
(86) Internationale Anmeldenummer: PCT/EP2014/068168
(87) Internationale Veröffentlichungsnummer: WO 2015/028506

(56) Entgegenhaltungen:
- DE-A1- 19 954 643
- DE-T2- 69 303 756
- US-A- 5 110 785

## Beschreibung

Die Erfindung betrifft poröse, schwimmfähige, biologisch abbaubare Ölbinder mit biologisch funktionalisierter Oberfläche zum beschleunigten Abbau bzw. zur Entfernung mineralölbasierter Verunreinigungen in Meeren, Flüssen, Binnengewässern sowie Rückhaltebecken oder Abwasserbehandlungsanlagen. Öl ist ein komplexes Stoffgemisch aus organischen Verbindungen: Aromaten, Alkenen, Alkanen, Paraffinen, Naphtenen sowie Einzelelementen wie Stickstoff, Sauerstoff, Schwefel, Natrium, Nickel, Eisen, Vanadium und anderen. Transportiert wird es zumeist als Rohöl. Häufig werden jedoch auch dessen Fraktionen wie Diesel-, Heiz- und Bunkeröle auf dem Seeweg transportiert. Bei der Bekämpfung von Bedrohungen durch ausgetretenes Öl liegen die Schwierigkeiten vor allem in der schnellen Ausbreitung und Vereinzelung der Ölschichten und der Vermischung von Öl und Wasser.

Durch Unfälle bei der Gewinnung und beim Transport von Öl bzw. Mineralölerzeugnissen auf See und der Verarbeitung von Öl kommt es immer wieder zur Verunreinigung von See- und Küstengebieten mit Öl. Der zähe Ölschlamm kann im Wasser ebenso wie an Land nur sehr langsam durch natürliche Biodegradation abgebaut werden.

Neben ökologischen Schäden entstehen bei einem Austritt von Öl auf See aber auch ökonomische Schäden in Form von Reinigungskosten, Produktionsverlusten, Einnahmerückgang in Fischerei und Tourismus sowie Strafverfolgungskosten. Die zeitnahe und kostengünstige Beseitigung von Verunreinigungen durch Öl in Seegebieten ist daher eine wichtige Aufgabe.

Derzeit werden unterschiedliche Systeme zur Beseitigung von Ölverschmutzungen auf See vorgehalten und eingesetzt, deren Effektivität von einer Vielzahl von Faktoren abhängig ist. Dies sind in erster Linie Ölcharakteristika, Wetterbedingungen, Wassertemperatur, Salinität, Wassertiefe und Erreichbarkeit des Seegebiets mit technischen Geräten aber auch die Entfernung zu sensiblen Ökosystemen bzw. vom Menschen genutzten Küstenabschnitten. Folgende Verfahren zur Beseitigung von Ölverschmutzungen auf See können grundsätzlich eingesetzt werden:
- Sammlung von Öl unter Verwendung mechanisch-physikalischer Verfahren
- Einsatz chemischer Mittel zur Veränderung der Ölcharakteristika und zur Verteilung des Öls im Wasserkörper (Dispergieren)
- Einsatz von Düngemitteln zur Unterstützung der natürlichen Biodegradation
- Thermische Behandlung des ausgetretenen Öls
- Reinigung der Küstenlinie unter Einsatz mechanisch-physikalischer Verfahren

Die vorhandenen Technologien zur Aufnahme von Öl auf See weisen eine eingeschränkte Einsatzfähigkeit und Wirksamkeit bei ungünstigen Witterungs-, Seegangs- und Strömungsbedingungen auf. Insbesondere die Beseitigung von Öl in Seegebieten mit geringen Wassertiefen und in küstennahen Bereichen ist durch den Tiefgang vieler Ölbekämpfungsschiffe und durch die vergleichsweise kurze zur Verfügung stehende Zeit ein weiteres Problem. Oft sind die küstennahen Flachwassergebiete durch eine erhöhte ökologische Sensibilität gekennzeichnet. Der Einsatz thermischer bzw. chemischer Verfahren ist in diesen Bereichen nicht möglich.

Das zur Ölbekämpfung im Küstenbereich vorgehaltene System besteht aus Spezialschiffen, mobilen Bekämpfungsgeräten wie Ölsperren, Leichterungssystemen, Hochdruckreinigern, Skimmern u.ä. sowie der Flugüberwachung zum Erkennen und zur Beobachtung von Ölverschmutzungen. Die in den Küstengewässern im Einsatz befindlichen Spezialschiffe weisen spezielle Einrichtungen zum Eingrenzen und Abschöpfen von Öl von der Wasseroberfläche und zur Aufnahme des Öls in auf den Schiffen vorgehaltenen Tanks auf.

Einer der wichtigsten Gesichtspunkte bei der Bekämpfung von Ölunfällen ist der zeitnahe Einsatz von effektiven Bekämpfungsmitteln nach einer Havarie, um eine großflächige Ausbreitung der Verschmutzung und damit die Gefährdung großer Lebensräume sowie touristisch und industriell genutzter Küstenbereiche zu vermeiden. Zudem kann oft nur eine zeitnahe Reaktion zu einer weitgehenden Beseitigung der Verschmutzung führen, da die Öleigenschaften nach Austritt auf die Wasseroberfläche einer schnellen Änderung unterliegen. Durch Evaporation der leichten Bestandteile formt das Öl nach kurzer Zeit einen klebrigen, festen Schlick, der über einen langen Zeitraum schwimmend im Wasser verbleiben kann oder Klumpen bildet, die auf den Meeresboden absinken. Der zähe Ölschlamm kann im Wasser ebenso wie an Land nur sehr langsam durch natürliche Biodegradation abgebaut werden.

Mit den weltweit im Einsatz befindlichen Systemen ist ein rasches und ökologisch sinnvolles Eingreifen in einer Vielzahl von Havariefällen nicht möglich. Einschränkungen ergeben sich insbesondere bei ungünstigen meteorologischen und hydrologischen Bedingungen sowie in Flachwasserbereichen und küstennahen Gebieten. Häufig vergehen bereits lediglich für den schiffsgebundenen Transport der Ölhavariebekämpfungssysteme vom Stationierungs- zum Unglücksort mehrere Tage. Hieran schließen sich oftmals weitere Verzögerungen infolge ungünstiger Witterungsbedingungen an, denn der Einsatz der bisherigen Reinigungssysteme erfordert eine verhältnismäßig ruhige See. Somit können Maßnahmen zur Reinigung vielfach erst Tage und Wochen nach Austritt des Öls begonnen werden. Zusammenfassend ist festzustellen, dass mit den bestehenden Systemen zur Reinigung von Ölverschmutzungen auf See in den meisten praktischen Fällen nur unzureichende Reinigungsraten erzielt werden können. Selbst unter optimalen Einsatzbedingungen können die bisher verfügbaren mechanischen Reinigungssysteme lediglich Reinigungsraten von rund 15 % erreichen, während Raten von unter 10 % die Regel darstellen.

Ölbinder werden eingesetzt, um ausgetretenes Öl zu ad-/absorbieren. Dadurch können Schäden bzw. Gefahren für Mensch und Umwelt reduziert werden. Das ausgetretene Öl kann in Kombination mit Ölbindern auch einfacher aufgenommen und entsorgt werden. Der Einsatz von oleophilen, hydrophoben Bindermaterialien zur Ölschadensbekämpfung ist nicht neu. Es wird zwischen aktivem und passivem Einsatz der Bindemittel unterschieden. Bei den aktiven Verfahren werden die Bindemittel z.B. schiffsgebunden in die Ölverschmutzung eingebracht und direkt wieder aus dem Gewässer entnommen (Skimmer, Ölsperren, Endlosfaserbündel). Die Binder stehen bei diesen Verfahren im ständigen Kontakt mit dem Schiff oder einem anderen Trägerfahrzeug.

Bei den passiven Bekämpfungsverfahren werden die Bindemittel als Granulat, Vlies oder Schwimmsperre aus der Luft oder vom Schiff aus in die Verschmutzung eingebracht, dort freigesetzt und erst nach einiger Zeit wieder aus dem Gewässer entnommen. Beim passiven Einsatz von Ölbindemitteln in Gewässern sind die Binder ohne und mit Ölbenetzung schwimmfähig und so beschaffen, dass sie möglichst schwimmend zu bergen sind.

Auf dem Markt wird eine Vielzahl unterschiedlicher mineralischer und organischer Materialien für die Verwendung als Ölbinder angeboten. Diese sind in der "Liste der geprüften Ölbindemittel Typ I, II, III und IV" aufgeführt, die durch den Verband der Hersteller geprüfter Öl- und Chemikalienbindemittel (GÖC e.V.) im April 2013 veröffentlicht wurde.

Untersuchungen zum Einsatz von passiven Bindern in der Meeresumgebung zeigen, dass die Wiederaufnahme von ausgebrachten Bindern ein noch nicht zufriedenstellend gelöstes Problem darstellt und deswegen auf die Verwendung von Streu- und Vliesgut bei Ölunfällen auf See verzichtet wird (OEBIUS, H. (2002): "Ölschadensbekämpfung auf Gewässern". GMAG Seminar "Bindemittel", Fachausschuss "Gerätschaften und Mittel zur Abwehr von Gewässergefährdungen (GMAG)").

Ein ungelöstes Problem beim Einsatz von Bindern ist der Eintrag von weiteren nicht bzw. nur sehr langsam abbaubaren Stoffen in das Meer. Während der Abbauprozesse entstehen z.T. toxisch wirkende Stoffe. Bei der Bergung von ausgebrachten Bindern wird abhängig von den meteorologischen und hydrodynamischen Bedingungen und der eingesetzten Bergetechnik immer nur ein Teil der ausgebrachten Binder aufgenommen werden können.

Als Beispiel ist in Koppe et al. (KOPPE, B.; KOHLHASE, S.; SCHULZ-BULL, D.; JÜRGENS, M.W. (2003): "SORBMOP - Clean-Up Technology for Oil Spills". Proc. 6th Conference on Coastal and Port E) ein Ölhavariebekämpfungssystem beschrieben, bei dem Kunststoffbinder aus hydrophobem und oleophilem Polyurethan-Kunststoffmaterial mit hoher Adsorptionsrate eingebracht werden.

Der Einsatz von schwimmenden Schaumstoffelementen ist auch in Druckschrift DE 100 39 875 A1 beschrieben. Diese werden durch Schiffe oder Flugzeuge ausgebracht und mit Hilfe von Netzen wieder eingesammelt und verbrannt. Die Ausbringung nicht biologisch abbaubarer Ölbindersysteme ist aber von behördlicher Seite aus Umweltschutzgründen in vielen Meeresgebieten und insbesondere auf der Ostsee nicht zugelassen.

DE 102 48 539 A1 offenbart eine Absorptionsmatte zur Aufnahme von Flüssigkeiten, vorzugsweise von Ölen oder ähnlich Stoffen von flüssigen Medien oder festem Untergrund, die dadurch gekennzeichnet ist, dass zwischen zwei fluiddurchlässigen Textilbahnen, wie einem Nähwirkvliesstoff, einem Nadelvliesstoff, Gewebe, Gewirke oder Verbundvliesstoff, eine Zwischenschicht aus einem biologisch abbaubarem Absorptionsmaterial angeordnet ist, wobei die Textilbahnen durch nahtartige Verbindungen miteinander verbunden sind und das Absorptionsmaterial zwischen den Verbindungsnähten von den Vliesstoffbahnen stabil eingeschlossen ist.

Das Absorptionsmaterial wird von einem mit ölabbauenden Mikroorganismen geimpften organischen Trägermaterial aus Leder in Form von Fasern, Granulat, Presslingen oder anderen riesel- bzw. schüttfähigen Formen gebildet, dass eine schnelle und vollständige Aufnahme von Öl bzw. ölhaltigen Schadstoffen und gleichzeitig den Abbau der aufgenommenen Schadstoffe in der Matte noch während des Einsatzes bzw. bei deren Bergung und Lagerung garantiert.

Das absorbierende Textil ist vorzugsweise für die Prävention von ölhaltigen Verschmutzungen vorgesehen, dass einer mehrfachen Wiederverwendung zugeführt werden kann und das schnell und problemlos transportiert und am Einsatzort verlegt werden kann und das in ausreichender Menge und entsprechenden Abmessungen zur Verfügung steht.

Nachteilig an dieser Absorptionsmatte ist der vergleichsweise komplizierte Aufbau aus mehreren funktionellen Schichten, wobei mehreren Textilbahnen, die das Absorptionsmittel mechanisch stabil einschließen und durch nahtartige Verbindungen miteinander verknüpft sind.

DE 102 44 122 C1 offenbart eine Ölbindematte zur Eingrenzung und/oder Beseitigung von Kontaminationen wie Erdölen, synthetischen Ölen, Schmierstoffen, Brennstoffen, Mineralölen, u.ä. Kohlenwasserstoffen oder Kohlenwasserstoffgemischen, bevorzugt auf Wasseroberflächen oder Oberflächen fester Böden aus organischem Bindemittel mit flächiger Umhüllung.

Dabei ist das hydrophobierte Bindemittel in der flexiblen, für hydrophobe Flüssigkeiten durchlässigen textilen Umhüllung aus Baumwollgewebe, Gewebe, Gestricke, Vliese und anderen Materialien angeordnet.

Die flächige Umhüllung enthält eine Beschichtung auf Siliconbasis, wodurch sie hydrophobiert ist und für hydrophile Flüssigkeiten, wasserlösliche Substanzen und das Bindemittel undurchlässig aber für lipophile Substanzen durchlässig ist.

Die Vorteile des Ölbinders ergeben sich aus dem besonderen Verhältnis von Mattenoberfläche zum Bindemittel. Das Gewichtsverhältnis Bindemittel zur Umhüllung beträgt 5:1 bis 25:1, wobei das Bindemittel eine Körnung bis zu 4,3 mm, ein Schüttgewicht von 0,2-0,7 g/cm³ bei einer Aufnahmekapazität von 0,15-0,75 I Heizöl/l Bindemittel aufweist. Das Bindemittel enthält Braunkohlegranulat und das Flächengewicht der Umhüllung beträgt gleichzeitig 250 bis 350 g/m³. Besonders vorteilhaft ist die Verwendung von einem Gemisch aus einer ausgewählten Braunkohlefraktion und naturbelassener Baumrinde oder auch von Materialien wie Sägespänen, Torf und Pflanzenfasern. Durch Beimischung der Rinde wird das Flächengewicht verringert und die Entflammbarkeitstemperatur herunter gesetzt.

Die Matten, insbesondere die darin enthaltenen Bindemittel können auch zusätzlich mit Mikroorganismen belegt werden. Dabei handelt es sich um Organismen der Gattungen Pseudomonas, Bacillus, Mesorhizobium und Pseudaminobacter.

Es hat sich gezeigt, dass die Bindemittel bezüglich der Ölaufnahme erst durch den Einschluss in die wasserabweisende/öldurchlässige textile Umhüllung ihre volle Wirksamkeit erreichen, da bei einem vorherigem Kontakt mit Wasser, der beim Einsatz häufig auftritt, die Aufnahmekapazität der Bindemittel (z.B. der Braunkohle) für ölartige Flüssigkeiten sonst stark zurückgeht.

Ohne die Textilhülle ist zudem die lange Schwimmfähigkeit nicht gegeben. Die Schwimmfähigkeit der Matten kann zusätzlich durch Anbringen von Schwimmkörpern aus Holz, Styropor oder ähnlichen erhöht werden, wodurch die Erfindung nicht eingeschränkt wird.

Ein problemloses und kontrolliertes Ausbringen und vor allem Wiederaufnehmen, auch bei ungünstiger Witterung, ist ebenfalls erst durch die textile Umhüllung möglich. Die Ölbindematten werden dabei von Schiffen zum Ort des Ölschadens transportiert und dort auf der Wasseroberfläche aufgebracht (z.B. abgerollt bzw. ausgelegt).

Nachteilig an dieser Ölbindematte ist der vergleichsweise komplizierte Aufbau aus einer textilen Umhüllung, die mit einem säure-, öl- und wasserbeständigem Polyestergarn gefertigt ist und außerdem das organische Bindematerial einschließt und zusätzlich mit Schwimmkörpern versehen werden kann. Außerdem wir die Schwimmfähigkeit der Ölbindematte erst durch die hydrophobierte Textilhülle möglich. Des Weiteren können die Ölbindematten nur mit dem Schiff zum Einsatzort transportiert und ausgebracht werden.

DE 20 2010 003 238 U1 offenbart eine Vorrichtung zur Aufnahme und/oder Eingrenzung von nicht mit Wasser mischbaren Flüssigkeiten wie Erdölen, synthetischen Ölen, Schmierstoffen, Brennstoffen, Mineralölen, Kohlenwasserstoffen oder Kohlenwasserstoffgemischen, bevorzugt von Wasseroberflächen oder von Oberflächen fester Böden.

Dabei ist ein Braunkohle enthaltendes Bindemittel in einer Umhüllung aus natürlichem Stoff oder Kunststoff, insbesondere Wolle, Baumwolle, Polyester, Polyethylen, Polypropylen oder anderen Polyolefinen eingebracht. Die Umhüllung weist eine hydrophobe Oberflächenmodifizierung auf, insbesondere in Form einer Silikonisierung, die durch Tränken, Einpinseln oder Aufsprühen auf die äußere oder jede einzelne Lage der Umhüllung aufgebracht wird, wodurch diese für hydrophile Flüssigkeiten und das Bindemittel undurchlässig ist. Die Umhüllung kann insgesamt flächig oder walzenförmig ausgestaltet sein und eine umlaufende Randverstärkung, insbesondere in Form eines Gewebebandes aufweisen. Beispiel für eine flächige Ausgestaltung ist die Bildung von Kissen oder Matten. Außerdem weist die Umhüllung wenigsten zwei miteinander verbundene oder getrennte Kammern in vorzugsweise kassettenartiger oder paralleler Anordnung auf, die mit Bindemittel gefüllt sind.

Das Bindemittel besteht zu wenigstens 50 Gew.-% vorzugsweise zu wenigstens 80 Gew.-% besonders bevorzugt vollständig aus Braunkohlenkoks. Bei ausschließlicher Verwendung von Braunkohlenkoks als Bindemittel können Ölaufnahmen aus Wasser in Größenordnungen von 0,3 bis nahezu 1 Liter Öl pro Liter Bindemittel erzielt werden. Das Bindemittel kann weitere Bestandteile wie Braunkohle, Holzkohle, Aktivkohle, Baumrindengranulat, Holzgranulat oder - späne, Torf, Pflanzenfasern und/oder mineralische Bindemittel wie beispielsweise Tonerde oder andere silikatische Materialien wie pyrogene Kieselsäure oder Fällungskieselsäure enthalten. Die Vorrichtung enthält keine zusätzlichen Schwimm- oder Auftriebseinrichtungen.

Nachteilig an dieser Vorrichtung ist der vergleichsweise komplizierte Aufbau aus einer Umhüllung, bevorzugt aus zwei oder drei Lagen, die mit Kammern versehen sind.

US 7 655 149 B1 offenbart ölabsorbierende Kenafbälle, bei denen Kenaffasern verwickelt werden, um Kugeln zu erhalten, die sich als sehr nützlich bei der Absorption von Öl und anderen organischen Flüssigkeiten an Land oder Wasser eignen.

Das Erzeugnis kann dabei verschiedene Formen annehmen, wie kugelförmig, länglich, ballförmig, konisch, zylindrisch u.a. Die Kenafkugeln können für einige Anwendungen zusammen gebunden werden, um so eine mattenartige, gerollte, deckenartige, sackartige oder seilartige Anordnungen zu erhalten. Außerdem können sie in eine Decke oder ein Tuch gewoben werden, um kleinere ölverschmutzte Bereiche säubern zu können. Die Kenafkugeln können auch zur Beseitigung von Ölen auf der Wasseroberfläche eingesetzt werden. Sie können u.a. Rohöl, Motoröl, Leichtöl, Getriebeöl aber auch Pflanzenöle aufnehmen. Die Kenafkugeln sind wasserabweisend und können auch über längere Zeit auf dem Wasser schwimmen. Sie haben eine wesentlich höhere Affinität Öl aufzunehmen, als Wasser. Die Kenafkugeln können Öl in einer Menge von mehr als 1000% ihres Eigengewichtes, in einigen Fällen auch mehr als 1800% des Eigengewichtes aufnehmen. Die Kugeln haben eine Dichte im Bereich von ca. 0,02 g/cm³ bis ca. 0.15 g/ cm³ und eine Masse von 0,2 g bis 10g.

Nachteilig an den ölabsorbierenden Kenafbällen ist, dass ein Austrag mit Hilfe eines Flugzeuges auf die Wasseroberfläche bei rauem Wetter aufgrund ihrer geringen Masse als nicht möglich erscheint. Außerdem eher nachteilig für die Aufnahme dünnflächiger Ölschichten von der Wasseroberfläche ist die Kugelform der Kenafbälle, da nur ein geringer Teil der Kugeloberfläche vom Öl benetzt wird. Ein Einfangen mit Netztechnik würde außerdem sehr geringe Netzmaschenweiten erfordern.

DE 103 34 967 A1 offenbart einen schwimmfähigen Ölabsorber zum entfernen ölhaltiger Verschmutzungen auf Wasseroberflächen, bei dem ein Absorptionsmittel aus aufgeschlossenen schwimmfähigen Holzfasern in Form von Spänen, Sägemehl, Schnitzeln oder Fasern besteht. Die Ölbindefähigkeit verbessert sich noch erheblich, wenn gemäß der bevorzugten Ausführungsform des Ölabsorbers den Holzfasern zerkleinerte Stengel von schwimmfähigen nachwachsenden Rohstoffen, wie Juncus effusus (Flatterbinse), Scirpus Lacustris (Teichsimse) oder anderen Pflanzen, die in den Zellzwischenräumen des Aerenchyms Lufteinschlüsse besitzen, als Kernfüllung beigemischt sind.

Das Absorptionsmaterial ist zu einem Strang geformt und von einer netzartigen Hülle aus einer großen Maschenstruktur aus Fäden, Folienbändchen oder dergleichen umgeben. In Strangrichtung parallel verläuft ein Zugelement, das aus einem schwimmfähigen wasserabweisenden Faserstoff besteht. Die Erfindung ist dadurch gekennzeichnet, dass mehrere schwimmfähige Stränge an den Strangenden, die von der netzartigen Hülle fest umschlossen werden und kein Absorptionsmaterial enthalten, in Längsrichtung miteinander verbunden sind. Die flexiblen Stränge können auch mattenartig miteinander verbunden sein, wobei Größe und Dicke der Matte entsprechend dem Verwendungszweck variierbar sind.

Nachteilig an diesem schwimmfähigen Ölabsorber ist der vergleichsweise komplizierte Aufbau, bei dem der schwimmfähige Ölabsorber aus einer netzartigen Hülle, die das Absorptionsmittel beinhaltet und dieses zu einem Strang formt, in dem parallel ein Zugelement zum Verbinden der Stränge enthalten ist, besteht.

DE 103 03 198 A1 offenbart ein Verfahren zum Aufsaugen von Öl vom Wasser. Durch hydrophobe Mittel, die in Jute, Filtervlies, Netze sowie andere durchlässige Gewebe verpackt sind, wird auf dem Wasser schwimmendes Öl aufgesaugt. Die Elemente können in Form von Säcken, Schläuchen oder Matten in allen benötigten Größen, Breiten und Längen hergestellt werden. Das leichte hydrophobe Material besteht aus Wärmedämmmatten oder Granulat und ist mit flüssigem Silicon, Siloxan oder anderen hydrophoben Mitteln getränkt. Sie können bei jedem Seegang sowie von allen Schiffstypen und Schiffsgrößen über das Wasser geschleppt werden.

Nachteilig an diesem Verfahren ist der vergleichsweise komplizierte Aufbau, bei dem die hydrophoben Wärmedämmmatten oder das Granulat erst in ein anderes Material verpackt werden und diese von Schiffen gezogen werden müssen.

DE 101 11 638 A1 offenbart ein Mittel und ein Verfahren zur Aufnahme von Chemikalien, insbesondere zur Aufnahme von Ölschichten, die auf Wasseroberflächen schwimmen.

Es wird ein Bindemittel zur Aufnahme von Chemikalien, insbesondere von auf der Wasseroberfläche schwimmenden Ölschichten vorgeschlagen, das aus einem faserförmigen Material besteht, das gefilzt oder gewebt ist, wobei aus dem gefilzten oder gewebten faserförmigen Material ein mattenartiger Körper gebildet wird.

Das faserförmige Material ist vorzugsweise ein Naturstoff, der einen hohen Kohlenstoffanteil aufweisen sollte. Als faserförmiges Material wird Xylit bevorzugt, da es ein umweltfreundlicher Naturstoff ist, der bei der Braunkohleverwertung und -verarbeitung anfällt. Anstelle des Xylit können auch Naturfasern als faserförmiges Material eingesetzt werden, bevorzugt sind Naturfasern, die aus der Gruppe ausgewählt sind, die aus Hanf, Rapsstroh, Holzfasern, Schilf, Maispflanzen und Flachs bestehen. Die verfilzten oder verwobenen Fasern sind zweckmäßigerweise getrocknet und/oder verkokt. Der so erhaltene mattenartige Körper wird auf die aufzunehmende Chemikalie aufgelegt und nach der Bindung der Chemikalie in den mattenartigen Körper wieder entfernt.

Nachteilig an diesem Bindemittel zur Aufnahme von Chemikalien ist, dass das faserförmige Material verfilzt oder gewoben werden und das Bindemittel nach der Aufnahme wieder vom Einsatzort entfernt werden muß.

DE 196 28 751 A1 offenbart ein Schwimmgut und dessen Herstellung, das in der Lage ist, auf Wasseroberflächen schwimmende Öle und Fette aufzunehmen. Es ist insbesondere für die Aufnahme von schweren und leichten Mineralölen, Erdölen, tierischen und pflanzlichen Ölen und Fetten sowie von Motorkraftstoffen vorgesehen.

Das Schwimmgut besteht aus zerkleinerten und zerfaserten pflanzlichen oder biostämmigen gut schwimmenden Faserstoffen, mit einer Faserlänge von ≤ 15 mm, vorzugsweise 1-5 mm, die mit einem stabilen hydrophoben Schutzfilm aus Montanharz umhüllt sind.

Als Faserstoffe eignen sich alle gut schwimmfähigen Stoffe pflanzlicher oder biostämmiger Natur, wie beispielsweise Holzspäne, ausgewählte Stroh- oder Schilfspäne sowie gereinigter Braunkohlexylit. Die zerkleinerten Späne und Faserstoffe werden zunächst mit Wasser gesättigt und danach mit einem stabilen hydrophilen Film überzogen. Nachfolgend wird Wasser aus den Poren der Faserstoffe durch schonende Trocknung wieder entfernt. Durch den Trocknungsprozess wird die hydrophobe Schutzhülle nicht zerstört, vielmehr verteilt sich der Schutzfilm infolge der Erwärmung weiter und die Oberfläche der Fasern wird vollständig hydrophobiert.

Das wird durch den Einsatz von Montanharz mit besonders guten Hafteigenschaften und vorteilhaften Verteilungsvermögen auf der Faseroberfläche vor und während der Trocknung gesichert. Das gute Verteilungsvermögen ist durch Ausbildung eines dünnen Schutzfilmes ohne tiefes Eindringen in die Poren des Trägermaterials gekennzeichnet. Außerdem wurde erkannt, dass Montanharz aufgrund seiner chemischen Stoffgruppenzusammensetzung ein besonders geeigneter Haftvermittler für Öle und Fette an den beschriebenen Faserstoffen, z.B. an Holzspänen ist. Für das feste Anhaften des Montanharzes an den Fasern und für seine öl- und fettsammelnde Wirkung ist nach DE 196 28 751 A1 sein bifunktioneller Charakter, der durch das Vorhandensein von sowohl hydrophilen als auch hydrophoben funktionellen Molekülgruppen bedingt ist, von gewichtiger Bedeutung. Bekanntlich ist die Beschichtung von mit Wasser gesättigten Materialien mit heiß verflüssigtem Montanharz, mit einem Schmelzbereich von 75 bis 85°C, nicht möglich, weil heiße flüssige Produkte, wie Öle, Fette, Wachse und Harze nicht an feuchten Oberflächen haften und zudem schwierige Mischbedingungen durch Wasserdampfbildung auftreten. Nach DE 196 28 751 A1 mußte deshalb ein Hydrophobierungsstoff gefunden werden, der eine stabile Haftung an der feuchten Oberfläche des Trägermaterials weit unterhalb seiner Schmelztemperatur ermöglicht, und der außerdem gewährleistet, dass die Schutzschicht bei der Trocknung erhalten bleibt. Die mit Montanharz konditionierten Faserstoffe haben einen Feuchtegehalt von etwa 5 bis 20%

Nachteilig an diesem Schwimmgut ist, dass die Hydrophobierung der Faseroberfläche auf den Einsatz von Montanharz beschränkt ist. Des Weiteren werden nur lose Faserstoffe und/oder Späne eingesetzt, die sich nur schwer wieder aus dem Wasser entfernen lassen. Zudem müssen die nach der Ölaufnahme entstandenen Agglomeratstrukturen zum Beispiel durch Abschöpfen oder Abkämmen eingesammelt werden, bevor diese auf den Gewässergrund absinken.

DE 2212 605 A1 offenbart ein Verfahren zur Beseitigung öliger Verschmutzungen, wie Ölflecken von Gewässern, unter Benutzung hydrophobierter feinzerteilter Holzpulpe. Dabei erfolgt die Hydrophobierung der Holzpulpe durch Behandeln mit einem synthetischen oder natürlichen Leimungsmittel, wie einem Harzleim, einer Wachsemulsion, einer Emulsion eines dimeren Alkylketens, einer Stearinsäureanhydridemulsion oder eines anderen natürlichen oder synthetischen Leimungsmittels, und wenn Harzleim verwendet wird, auch mit Alaun, Aluminiumchlorid, Natriumaluminat, einem wasserlöslichen Aluminiumsalz oder einem Chrom-, Erdalkali-, Eisen- oder Mangan-Salz. Die Flocken dienen zur Entfernung von Öl von der Wasseroberfläche und werden anschließend zu Briketts verpresst.

Nachteilig ist hier die geringe Dichte und Festigkeit und die flockige Form des Bindermaterials, die eine Ausbringung mit dem Flugzeug und eine Bergung mit Netzen nicht erlauben.

AT 347 362 B offenbart ein Mittel auf Basis von Zellulose bzw. Holzfasern zum Aufnehmen und/oder Binden von insbesondere umweltgefährdenden Flüssigkeiten und ein Verfahren zur Herstellung. Dabei werden als Rohstoffe Spuckstoff ("Rejekt") von Sortier- und Cleaneranlagen und/oder Schlamm aus Abwasseranlagen der Papier- und/oder Zellstoff-fabrikation und gegebenenfalls "Äste"-Stoff wie er als Abfall bei der Zellstofffabrikation anfällt eingesetzt, wobei diese Abfallstoffe gegebenenfalls mit einem Hydrophobierungsmittel hydrophobiert sind. Diese werden in granulierter bzw. pelletierter Form als Ölbinder eingesetzt.

Nachteilig an diesem Bindemittel ist die Verwendung von Spuckstoff aus der Papierindustrie, der hohe Anteile an Kunststoffen und Metall und nur geringe Holzfaseranteile besitzt. Auch die Pelletform der Binder ist nachteilig.
JP S5276287 A offenbart ein Ölbindematerial bestehend aus Holzspänen, die mit einer Paraffin-Wachs Emulsion, einem Zirconium Salz und mit einem Phenolharz imprägniert sind.

Nachteilig ist hier der Einsatz von Ölbindern in Form von Holzspänen.

Der biologische Abbau vieler, in Mineralölen enthaltener Kohlenwasserstoffe durch Mikroorganismen ist in vielfältigen terrestrischen und marinen Ökosystemen, wie in Böden und dem Meereswasser beschrieben worden (DELILLE D., BASSERES A., DESSOMMESS A. (1998): Effectiveness of bioremediation for oil-polluted Antarctic seawater. Polar Biol 19:237-241; DELILLE D., DELILLE B. (2000): Field observations on the variability of crude oil impact in indigenous hydrocarbon-degrading bacteria from sub-Antarctic intertidal sediments. Mar Environ Res 49:403-417; SIRON R., PELLETIER E., BROCHU C. (1995): Environmental factors influencing the biodegradation of petroleum hydrocarbons in cold seawater. Arch Environ Contam Toxicol 28:406-416).

Bei der Bioremediation kontaminierter Ökosysteme werden bevorzugt Bakterien (STEPHEN J.R., MACNAUGHTON S.J. (1999): "Developments in terrestrial bacterial remediation of metals). Curr. Opin. Biotechnol. 10 (3), 230-233) und Pilze (COULIBALY L., GOURENE G., AGATHOS N.S. (2003): "Utilization of fungi for biotreatment of raw wastewaters". Afr. J. Biotechnol. 2 (12), 620-630) eingesetzt. Im letzten Jahrzehnt wurde der Bioremediation mittels Pflanzen, d.h. der Phytoremediation größere Aufmerksamkeit geschenkt (MACHATE T., NOLL H., BEHRENS H., A. KETTRUP A. (1997): "Degradation of phenanthrene and hydraulic characteristics in a constructed wetland". Wat. Res. 31 (3), 554-560). Algen werden häufig bei der Säuberung von landwirtschaftlichen und kommunalen Abwässern eingesetzt (OSWALD, W. J. (1995), Ponds in the twenty-first century. Wat. Sci Tech, vol. 31, No. 12, pp. 1-8). Bei der Beseitigung von Ölen und polyzyklischen Kohlenwasserstoffen sind diese Organismen bisher noch nicht eingesetzt worden.

Es ist bekannt, dass Assoziationen bestehend aus verschiedenen Organismen bevorzugt für die Bioremediation verwendet werden, da es praktisch keinen Organismus gibt, der befähigt ist, alle Komponenten einer solch komplexen Verunreinigung abzubauen (OSTWALD, W. J., (1995), Ponds in the twenty-first century. Wat. Sci Tech, vol. 31, No. 12, pp. 1-8).
Im stationären Einsatz an Land wurden phototrophe Partner wie eukaryotische Algen oder Cyanobakterien in Kombination mit heterotrophen Partnern wie z.B. alkanotrophe Bakterien bei der Reinigung von industriellen Abwässern untersucht, wobei auch Ölrückstände abgebaut wurden (SAFONOVA E., KVITKO K.V., IANKEVITCH M.1, SURGKO L.F., AFTI I.A., REISSER W. (2004) Biotreatment of industrial wastewater by selected algal-bacterial consortia // Engineering in Life Sciences,. V. 4. P. 347-353). Bei diesen Untersuchungen des biogenen Ölabbaus konnte nachgewiesen werden, dass die Bioremediation durch Zugabe von phototrophen Partnern wie eukaryotischen Algen und Cyanobakterien beschleunigt wird (SAFONOVA E.TH., DMITRIEVA I.A.AND KVITKO K.V. (1999): The interaction of algae with alcanotrophic bacteria in black oil decomposition. In: Resources, Conservation and Recycling 27, p.193-201.).

DE 693 03 756 T2 offenbart poröse Ölbinder in Form einer latex-verbondeten Holzbrei aufweisenden Gewebestruktur, welche als Vlies aus lignocellulosehaltigen Rohstoffen aufgefasst wird. Die Ölbinder sind aus mehreren Schichten gefaltet.

Die Aufgabe der Erfindung betrifft die Entwicklung biologisch abbaubarer freischwimmender poröser Ölbinder in Form eines Vlieses zum beschleunigten Abbau bzw. zur Entfernung von Ölverschmutzungen von der Wasseroberfläche in Meeren, Flüssen, Binnengewässern sowie Rückhaltebecken oder Abwasserbehandlungsanlagen. Dabei kommt der Form, Dimensionierung, Dichte und Festigkeit der Vliese bzw. Ölbinder, eine besondere Bedeutung zu. Beim Einsatz der Ölbinder ist es nicht zwangsläufig erforderlich alle ausgebrachten Ölbinder wieder aufzunehmen, da sie aus biogenen Stoffen bestehen und biologisch abbaubar sind. Die Ölbinder sollen vor allem schnell am Einsatzort sein, da die Ölausbreitung mit hoher Geschwindigkeit vonstattengeht. Außerdem soll die Dicke der Ölbinder so gering wie möglich sein, da die Ölschichtdicke schnell abnimmt und das Öl sich mit Wasser vermischt und herkömmliche Aufnahmetechniken daher nur einen geringen Wirkungsgrad haben. Das neue Bindersystem soll auch bei schlechten Wetterbedingungen auf Meeren und Binnengewässern und insbesondere in Flachwasserbereichen ausgebracht werden können. Zusätzlich muss es eine geeignete Rieselfähigkeit besitzen, da es auch mit dem Flugzeug ausgebracht werden soll. Außerdem müssen die Binder so dimensioniert sein, dass sie bei der Bergung mit Netzsystemen nicht durch die Maschen rutschen und eine ausreichende Festigkeit und Stabilität besitzen. Neben den biologisch abbaubaren Ölbindermaterialien sind ölabbauende

Mikroorganismen, die auf den Ölbindern immobilisiert werden, eine mögliche Komponente des erfindungsgemäßen Materials. Der Abbau des Öls ist insbesondere für die Binder wichtig, die nicht geborgen werden können und somit im Ökosystem verbleiben. Damit kann ein beschleunigter Abbau des Öls erreicht werden. Für diese Binder ist eine lange Schwimmfähigkeit von mehreren Tagen erforderlich.

Erfindungsgemäß wird die Aufgabe durch einen porösen Ölbinder in Form eines Vlieses, bestehend aus Fasern aus lignocellulosehaltigen Rohstoffen, gelöst. Der Ölbinder weist folgende Merkmale auf:
a) Die Dichte des Ölbinders beträgt 80 bis 300 kg/m³, besonders bevorzugt 220 bis 290 kg/m³.
b) Der Ölbinder ist 3 bis 6 mm, besonders bevorzugt 3,5 bis 4,5 mm dick.
c) Die Breitfläche des Ölbinders hat eine Dimension von 9 bis 200 cm², vorzugsweise eine Dimension von 25 bis 100 cm², besonders bevorzugt im Bereich von 25 bis 30 cm².
d) Der Porenanteil des Ölbinders beträgt 30 bis 96%, vorzugsweise 80 bis 90%, gemessen am Gesamtanteil des Ölbinders.
e) Die Biegefestigkeit des Ölbinders beträgt mindestens 1,5 N/mm².

Überraschend hat sich gezeigt, dass Ölbinder, die mit einem Bedeckungsgrad von mind. 10 % eingesetzt werden, einen Reinigungsgrad im Bereich von 10 bis 100 %, vorzugsweise von 80 % erreichen. Vorteilhaft liegt durch den erfindungsgemäßen Aufbau des Ölbinders die Ölaufnahme im Bereich von 100 bis 700 kg/m³, vorzugsweise im Bereich von 400 bis 600 kg/m³, wobei ein Ölbinder nach der Aufnahme von Öl aus der Umgebung vorteilhaft eine Schwimmfähigkeit auf der Wasseroberfläche von mindestens 3 Tagen, vorzugsweise von mindestens 7 Tagen aufweist. Weiterhin vorteilhaft erlaubt der erfindungsgemäße Ölbinder nach der Aufnahme von Öl aus der Umgebung, dass beim Wirken eines Überdrucks von bis zu 0,1 bar auf den Ölbinder kein Öl abgegeben wird. Die Haltefähigkeit des Öls im Ölbinder bei einem Überdruck von 0,1 bar wurde nach der dem Fachmann bekannten Richtlinie LTwS-Nr. 27 (Lagerung und Transport wassergefährdender Stoffe vom Beirat beim Bundesministerium für Umwelt, Naturschutz und Reaktorsicherheit; herausgegeben vom Umweltbundesamt) überprüft.

Öl im Sinne der Erfindung umfasst alle gängigen Ölsorten, wie beispielsweise Rohöl, Leichtöl und Schweröl.

Unter der Dichte des Ölbinders versteht der Fachmann die Masse des Ölbinders in kg bezogen auf sein Volumen in m³.

Die Dicke des Ölbinders bezieht sich auf die Länge der kürzesten Kante des Ölbinders.

Die Breitfläche des Ölbinders umfasst im Sinne der Erfindung die Fläche, die beim Einbringen des Ölbinder in das Wasser bzw. Öl mit diesen in Kontakt kommt.

Der Porenanteil des Ölbinders beschreibt das zur Verfügung stehende Volumen für die Ölaufnahme bezogen auf das Gesamtvolumen des Ölbinder und wurde nach der für den Fachmann gängigen DIN 51913 bestimmt. Der Porenanteil besteht erfindungsgemäß aus den Hohlräumen in den Lumen (von der Zellwand eingeschlossener Hohlraum einer Zelle) der Holzfasern bzw. Holzzellen und aus den Hohlräumen, die im Ölbinder zwischen den Holzfasern liegen. Das Volumen der Zwischenfaserhohlräume korreliert mit der Dichte des Ölbinders.

Die Ölbinder besitzen eine erhöhte Biegefestigkeit um den Beanspruchungen bei Ausbringung und Bergung zu genügen. Die Biegefestigkeit des Ölbinders wird nach der dem Fachmann bekannten DIN EN 310 bestimmt.

Die porösen Ölbinder in Form eines Vlieses können flächige Gebilde unterschiedlicher Form, wie beispielsweise mehreckig, rechteckig, quadratisch, rund oder oval, bevorzugt rechteckig sein.

Bevorzugt weisen die vorwiegend rechteckigen Gebilde eine Kantenlänge von 10 cm, besonders bevorzugt von 5 cm auf. Die Kantenlänge bezieht sich dabei auf die beiden längsten gleichlangen Kanten des rechteckigen Gebildes.

Zur Erfindung gehört auch ein poröser Ölbinder in Form eines Vlieses, bestehend aus hydrophobierten Fasern aus lignocellulosehaltigen Rohstoffen, die mit natürlichen und/oder naturidentischen Additiven als Hydrophobierungsmittel benetzt sind mit folgenden Merkmalen:
a) Die Dichte des Ölbinders liegt im Bereich von 80 bis 300 kg/m³, besonders bevorzugt im Bereich von 220 bis 290 kg/m³.
b) Die Dicke des Ölbinders liegt im Bereich von 3 bis 6 mm, besonders bevorzugt im Bereich von 3,5 bis 4,5 mm.
c) Die Breitfläche des Ölbinders hat eine Dimension von 9 bis 200 cm², vorzugsweise eine Dimension von 25 bis 100 cm², besonders bevorzugt im Bereich von 25 bis 30 cm².
d) Der Porenanteil des Ölbinders liegt im Bereich von 30 bis 96%, vorzugsweise im Bereich von 80 bis 90%, gemessen am Gesamtanteil des Ölbinders.
e) Die Biegefestigkeit des Ölbinders beträgt mindestens 1,5 N/mm².

Bevorzugt weist der Ölbinder hydrophobierte Fasern aus lignocellulosehaltigen Rohstoffen auf.

Vorzugsweise bestehen die lignocellulosehaltigen Rohstoffe aus Holz, Getreide-, Flachs-, Raps-, Reis- oder Baumwollstroh, Kokos, Bagasse, Bambus, Kork, Seegras, Baumrinde oder Mischungen aus diesen. Bevorzugt ist Seegras, das an Stränden als Schwemmgut anfällt. Besonders bevorzugt sind Nadelhölzer.

Die lignocellulosehaltigen Rohstoffe liegen im Ölbinder in Form von Fasern vor, welche im Sinne der Erfindung eine Länge von 0,1 bis 6 mm aufweisen. Bei Ölbindern aus Nadelholzvliesen weisen die Nadelholzfasern eine Länge von 0,5 bis 4,0 mm auf.

In einer besonderen Ausgestaltung der Erfindung sind die lignocellulosehaltigen Rohstoffe thermisch modifiziert. Dem Fachmann sind verschiedene Verfahren zur thermischen Modifikation von Holzfasern bekannt. Beispielsweise kann die Holzfaser oder das Holz vor der Mahlung unter Luftabschluss und/oder Stickstoffatmosphäre in einem Autoklav in einem Temperaturbereich von 160 bis 260 °C behandelt sind. Vorteilhaft kann die Holzfaser durch die thermische Modifikation weniger Wasser aufnehmen.

Bevorzugt beträgt der Schlankheitsgrad der Fasern 0,5 bis 5, besonders bevorzugt 1,0 bis 4, ganz besonders bevorzugt 1,5 bis 3, wobei besonders lange schlanke Fasern vorteilhaft sind, da diese die Festigkeit der Ölbinder positiv beeinflussen. Unter dem Schlankheitsgrad im Sinne der Erfindung versteht der Fachmann das Verhältnis von Länge der Fasern zum Durchmesser der Fasern (Lexikon der Holztechnik, 4. Auflage, Fachbuchverlag Leipzig, 1990, S. 640).

Der erfindungsgemäße Ölbinder besitzt variierende Porengrößen, wodurch vorteilhaft unterschiedliche Arten von Ölen aufgenommen werden können.

Des Weiteren ist vorteilhaft, dass der hohe Porenanteil zu einer großen spezifischen Oberfläche des Ölbinders führt, wodurch die durch Adhäsion verursachte Ölaufnahme beschleunigt wird. Vorzugsweise kann der Ölbinder tierische Rohstoffe mit einem Massenanteil im Bereich von 5 bis 15 Ma.-%, besonders bevorzugt im Bereich von 8 bis 12 Ma.-% aufweisen. Vorzugsweise sind die tierischen Rohstoffe Wolle, Federn oder Leder oder Mischungen aus diesen.

Die Kombination aus lignocellulosehaltigen Rohstoffen und tierischen Rohstoffen bewirkt eine verbesserte/beschleunigte Ölaufnahme in den Ölbinder. Besonders leichtflüchtige Fraktionen des Ölgemisches können durch die hohe Affinität der tierischen Rohstoffe schneller aufgenommen werden.

Nach einer besonderen Ausgestaltung der Erfindung sind die Fasern mit natürlichen oder naturidentischen Additiven wie Paraffine, Wachse, synthetisches oder natürliches Latex, Rindenextrakte, Tannine, besonders bevorzugt Gallusgerbsäure oder Mischungen aus diesen hydrophobiert. Bevorzugt sind die Tannine aus Holz und/oder Rinde eines Quebrachobaumes und/oder einer Eiche.

Überraschend hat sich gezeigt, dass die Ölaufnahme in den erfindungsgemäßen Ölbinder aufgrund der Hydrophobierung der Fasern sehr viel schneller als die Aufnahme des Wassers ist. Im bewegten Wasser wird bereits nach 10 bis 15 min bis zu 99 Ma.-% der Ölmenge aufgenommen, während die Wasseraufnahme mehrere Tage bis Wochen dauert. Weiterhin vorteilhaft ist die verbesserte Schwimmfähigkeit der mit Öl vollgesogenen Ölbinder durch die Hydrophobierung der Oberfläche der Fasern oder des Ölbinders.

Vorzugsweise ist die Faseroberfläche mit einem natürlichen oder synthetischen Bindemittel wie Stärke, Proteinen, Harnstoffharzen, Isocyanaten oder Mischungen aus diesen benetzt. Vorteilhaft wird durch das Bindemittel die Festigkeit des Ölbinders verbessert.

Vorzugsweise beträgt die Ausgleichsfeuchte des erfindungsgemäßen Ölbinder 5 bis 20 Ma.-%, besonders bevorzugt 8 bis 15 Ma.-%, gemessen an der atro Masse des Faserstoffes.

Dem Fachmann ist die Bezeichnung atro Masse des Feststoffes bekannt und bezeichnet die Trockenmasse des Feststoffes, nach dem dieser bis zur Massekonstanz getrocknet wurde.

Unter Ausgleichsfeuchte versteht der Fachmann die Feuchte, die der Ölbinder bei der Lagerung aufgrund des jeweils herrschenden Klimas annimmt.

Vorzugsweise weist die Oberfläche der Fasern oder des Ölbinders immobilisierte Mikroorganismen auf.

Vorzugsweise bestehen die Mikroorganismen aus Mikroorganismengemeinschaften aus alkanotrophen Bakterien der Gattungen *Rhodococcus, Pseudomonas und Sphingomonas* sowie phototrophen Algen und Cyanobakterien der Gattungen *Microcoleus, Phormidium, Lyngbya, Oscillatoria und Anabaena.*

In einer Vorzugsvariante wird eine Mikroorganismengemeinschaft bestehend aus ölabbauenden Mikroorganismen mit einem phototrophen Partner, z.B. Algen oder Cyanobakterien, eingesetzt. Diese bilden eine Biozönose, bei der die Algen molekularen Sauerstoff für die heterotrophen ölabbauenden Bakterien produzieren und so eine Sauerstofflimitation der Bakterien vermeiden. Vorzugsweise liegen dem erfindungsgemäßen Ölbinder lyophilisierte Mikroorganismen bei, die erst kurz vor dessen Einsatz in einer Flüssigkeit, vorzugsweise Wasser, suspendieren und anschließend auf den Ölbinder immobilisiert werden. Die schwimmfähigen porösen Ölbinder in Form eines Vlieses zum beschleunigten Abbau bzw. zur Entfernung von Ölverschmutzungen von der Wasseroberfläche in Meeren, Flüssen, Binnengewässern sowie Rückhaltebecken oder Abwasserbehandlungsanlagen werden durch ein Nassverfahren oder ein Trockenverfahren hergestellt.

Das Nassverfahren umfasst die Verfahrensschritte:
a) Erzeugen von Fasern aus lignocellulosehaltigen Rohstoffen durch thermische, hydrothermische, mechanische, thermomechanische oder chemische Aufschlussverfahren,
b) Vereinzeln und Suspendieren der Fasern in einer Mischbütte mit Wasser,
c) Zusetzen und Einmischen eines Hydrophobierungsmittels,
d) Formen, Entwässern und Trocknen eines Vlieses,
e) Formgebung der getrockneten Vliese,
wobei der Faseranteil in der Suspension bei 0,5 bis 4,5 Ma.-%, besonders bevorzugt bei 2,5 bis 3,5 Ma.-%, gemessen an der Gesamtmasse der Suspension, liegt. Es wird ein Vlies aus lignocellulosehaltigen Rohstoffen hergestellt. Die lignocellulosehaltigen Rohstoffe können durch thermische, hydrothermische, mechanische, thermomechanische oder chemische Aufschlussverfahren, wie Mahl- oder Schneidverfahren zu Fasern gemahlen werden.

Die Vorentwässerung der Suspension und die Formgebung des Vlieses erfolgt durch Einbringen in ein Sieb in einer gewünschten Größe. Durch Pressen wird das erhaltene Vlies weiter entwässert und auf die gewünschte Dicke und Dichte kalibriert und dann in einem Trockner getrocknet. Größere Vliese werden dann in kleinere Platten der gewünschten Größe aufgetrennt.

Vorteilhaft kann durch die Größe des Siebes und den Feststoffanteil in der Suspension die Dichte und die Biegefestigkeit der Ölbinder eingestellt werden. Dem Fachmann ist bekannt, wie der Feststoffanteil und Siebgröße ausgewählt werden müssen, um gezielt eine Dichte und eine Biegefestigkeit einzustellen.

Die Formgebung der getrockneten Vliese erfolgt nach dem Fachmann bekannten Methoden, beispielsweise durch schneiden oder sägen der Vliese in eine gewünschte Form.

Das Trockenverfahren umfasst folgende Verfahrensschritte:
a) Erzeugen von Fasern aus lignocellulosehaltigen Rohstoffen durch thermische, hydrothermische, mechanische, thermomechanische oder chemische Aufschlussverfahren,
b) Trocknen der Fasern,
c) Beleimen und/oder Benetzen der Fasern mit einem natürlichen oder synthetischen Bindemittel und/oder einem Hydrophobierungsmittel,
d) Formen eines Vlieses durch pneumatische oder manuelle Vliesstreuung der beleimten und/oder benetzten Fasern,
e) Pressen und Aushärten des Vlieses.
Es wird dabei ein Vlies aus lignocellulosehaltigen Rohstoffen hergestellt. Die lignocellulosehaltigen Rohstoffe werden durch thermische, hydrothermische, mechanische, thermomechanische oder chemische Aufschlussverfahren zu Fasern verarbeitet.

Die Fasern werden nach dem Aufschluss getrocknet.

Nach einer besonderen Ausgestaltung der Erfindung werden den Fasern natürliche oder naturidentische Additive wie Paraffine, Wachse, synthetisches oder natürliches Latex, Rindenextrakte, Tannine, besonders bevorzugt Gallusgerbsäure oder Mischungen aus diesen, zugegeben. Bevorzugt werden die Tannine aus Holz und/oder Rinde eines Quebrachobaumes und/oder einer Eiche zugegeben.

Den Fasern können nach dem Trocknen optional natürliche oder synthetische Bindemittel wie Stärke, Proteine, Harnstoffharze oder Isocyanate oder Mischungen aus diesen zugegeben werden.

Bevorzugt werden die natürlichen Bindemittel Stärke und Proteine mit einem Anteil von 2 bis 40 Ma.-%, besonders bevorzugt von 5 bis 30 Ma.-%, bezogen auf die atro Masse des Faserstoffes, zugegeben.

Bevorzugt werden Harnstoffharze mit einem Anteil von 5 bis 18 Ma.-%, besonders bevorzugt von 8 bis 12 Ma.-%, bezogen auf die atro Masse des Faserstoffes, zugegeben.

Bevorzugt werden Isocyanate mit einem Anteil von 1 bis 10 Ma.-%, besonders bevorzugt von 2 bis 8 Ma.-%, bezogen auf die atro Masse des Faserstoffes, zugegeben.

Durch pneumatische oder manuelle Vliesstreuung der beleimten und/oder benetzten Fasern auf eine gewünschte Größe wird ein Vlies geformt.

Das Vlies wird auf eine gewünschte Dicke gepresst und ausgehärtet.

Größere Vliese werden in kleinere flächige Gebilde verschiedener Formen wie beispielsweise vieleckig, rechteckig, quadratisch, rund oder oval und der gewünschten Größe aufgetrennt. Bevorzugt sind rechteckige Gebilde.

Bevorzugt werden die rechteckigen Gebilde mit einer Kantenlänge von 10 cm, besonders bevorzugt von 5 cm hergestellt. Die Kantenlänge bezieht sich dabei auf die beiden längsten Kanten des rechteckigen Gebildes.

Vorzugsweise werden als lignocellulosehaltige Rohstoffe Holz, Seegras, Baumrinde, Getreide-, Flachs-, Raps-, Reis- oder Baumwollstroh, Kokosfasern, Bagasse, Bambus, Kork oder Mischungen aus diesen eingesetzt. Bevorzugt ist der Einsatz von Seegras, das an Stränden als Schwemmgut anfällt. Besonders bevorzugt ist der Einsatz von Nadelhölzern.

Vorzugsweise können in den Ölbinder tierische Rohstoffe mit einem Massenanteil im Bereich von 10 und 15 Ma.-%, besonders bevorzugt im Bereich von 8 bis 12 Ma.-%, bezogen auf die Gesamtmasse des Ölbinders, eingesetzt werden. Vorzugsweise werden als tierische Rohstoffe Wolle, Federn oder Leder oder Mischungen aus diesen eingesetzt.

Bevorzugt werden die natürlichen Bindemittel wie Stärke und Proteine im Bereich von 2 bis 40 Ma.-%, besonders bevorzugt im Bereich von 5 bis 30 Ma.-%, bezogen auf die atro Masse des Faserstoffes, zugegeben.

Vorzugsweise werden an der Oberfläche der Fasern des hydrophobierten Vlieses durch Sprüh- und Tauchverfahren ölabbauende Mikroorganismen immobilisiert.

Vorzugsweise werden als ölabbauende Mikroorganismen alkanotrophe Bakterien der Gattung *Rhodococcus, Pseudomonas und Sphingomonas* sowie phototrophe Algen und Cyanobakterien der Gattungen *Microcoleus, Phormidium, Lyngbya, Oscillatoria und Anabaena* eingesetzt.

Die Immobilisierung erfolgt während der Herstellung oder vor Einsatz der Ölbinder.

Bevorzugt werden dem Ölbinder noch lyophilisierte oder in Suspension befindliche Mikroorganismen bei der Herstellung des Ölbinders und/oder vor dem Einsatz des Ölbinders zur Immobilisierung hinzugefügt.

Die gewonnen Binder dienen sowohl als Trägermaterial zur Immobilisierung von ölabbauenden Mikroorganismengemeinschaften als auch zur Aufnahme des Öls nach der Ausbringung. Für den beschleunigten Abbau des vom Trägermaterial absorbierten Öls werden während der Herstellung und/oder vor der Ausbringung des Ölbindematerials ölabbauende Mikroorganismengemeinschaften auf der Oberfläche und/oder in den Hohlräumen des Trägermaterials immobilisiert. Die Immobilisierung der Mikroorganismen kann während oder nach der Herstellung des Ölbinders oder erst kurz vor dem Einsatz erfolgen. Zum einen werden solche Ölbinder durch Tauchen des Vlieses in Mikroorganismen enthaltene Bäder oder durch Besprühen des Vlieses mit ölabbauenden Mikroorganismen hergestellt. Zum anderen können die vorgehaltenen Mikroorganismen, um eine längere Haltbarkeit (Überlebensfähigkeit) zu gewährleisten, diese kurz vor dem Havarieeinsatz in einer wässrigen Lösung suspendiert und appliziert werden.

Die erfindungsgemäßen Ölbinder werden zum Abbau bzw. zur Entfernung von Ölverschmutzungen von der Wasseroberfläche in Meeren, Flüssen, Binnengewässern sowie Rückhaltebecken oder Abwasserbehandlungsanlagen bereitgestellt.

Durch den Einsatz von biogenen, biologisch abbaubaren Ölbindern wird die Kontamination der Gewässer, in die der Binder ausgebracht wird, verhindert oder vermindert. Als Trägermaterial werden kostengünstige Reststoffe verwendet.

Die Ölbinder können schnell mit traditioneller Schiffs- und Fischereitechnik aber auch per Flugzeug im Bereich der im Wasser befindlichen Ölverschmutzung sowohl mit als auch ohne Mikroorganismen ausgebracht werden. Somit sind diese auch für den Einsatz in Gebieten mit geringer Wassertiefe sowie bei schwierigen Wetterbedingungen einsetzbar.

Die Ölbinder werden im Bereich der im Wasser befindlichen Ölverschmutzung mit einem Bedeckungsgrad von mind. 10 % verteilt. Überraschend dabei ist, dass die Ölaufnahme bis zur Sättigung aufgrund der geringen Dichte des Öls und des hohen Porenanteils der Binder innerhalb weniger Minuten erfolgt, während die Wasseraufnahme aufgrund der Hydrophobierung der Fasern sehr viel langsamer ist und mehrere Tage bis Wochen andauert. Dabei kann ein Reinigungsgrad der Wasseroberfläche von mehr als 80 % schon bei einem geringen Bedeckungsgrad von nur 10 % erreicht werden. Die Aufnahme des Öls durch das Ölbindermaterial verhindert eine weitere Verschmutzung des Gewässers und der Küste und vermindert die Gefährdung von Wasservögeln. Dabei hat der Ölbinder eine Schwimmfähigkeit auf der Wasseroberfläche von mehreren Tagen.
Nach der Ölaufnahme werden die beladenen Ölbinder unkompliziert mittels Netztechnik aus dem Wasser entfernt und der thermischen Verwertung zugeführt.

Bei beladenen Ölbindern die aufgrund ungünstiger Witterungsverhältnisse im Gewässer verbleiben oder an unzugänglichen Küstenabschnitten angespült werden, kann die Mikroorganismengemeinschaft ihre Wirkung entfalten. In einer Vorzugsvariante wird eine Mikroorganismengemeinschaft bestehend aus ölabbauenden Mikroorganismen mit einem phototrophen Partner, z.B. Algen oder Cyanobakterien, eingesetzt. Diese bilden eine Biozönose, bei der die Algen molekularen Sauerstoff für die heterotrophen ölabbauenden Bakterien produzieren und so eine Sauerstofflimitation der Bakterien vermieden wird.

Aufgrund des Wasser- bzw. Ölkontaktes wird das Wachstum stimuliert, die Mikroorganismen bewachsen den Ölfilm und beginnen mit dessen Abbau. Vorteilhaft ist es, wenn Mikroorganismen eingesetzt werden, die an die Umweltbedingungen des Einsatzortes angepasst sind. Die mit dem Ölbindematerial eingebrachte Mikroorganismengemeinschaft kann die Besiedlung und den Abbau der Ölverschmutzungen um mehrere Wochen und Monate beschleunigen. Der Abbau des chemisch unbedenklichen Trägermaterials erfolgt ebenfalls durch Mikroorganismen die sich nach oder während des Abbaus der toxischen Ölrückstände auf dem Trägermaterial ansiedeln. Im Ergebnis wird eine stark verminderte Belastung der Umwelt erreicht.

Anhand nachfolgender Ausführungsbeispiele wird die Erfindung näher erläutert.

### Beispiel 1a: Herstellung des Ölbindematerials mit Latex und Paraffin

Fichtenholzhackschnitzel wurden durch thermomechanische Mahlung zu Faserstoff verarbeitet und daraus in einer Mischbütte eine Suspension mit einem Feststoffanteil von 3 Ma.-% hergestellt. Der Stoffsuspension werden 7 % Latexmilch und 2 % Paraffin (Feststoff auf atro Faser) zugegeben und es wird bei 60 °C für ca. 20 min. gerührt. Als Fällungsmittel wird Al₂(SO₄)₂ zugegeben und die Suspension wird über ein Sieb mit einem Unterdruck von 0,8 bar entwässert. Das 4 mm dicke Vlies wird kalibriert und bei 180 °C bis auf einen Restfeuchtegehalt von 8 % getrocknet. Das produzierte Vlies besitzt eine Rohdichte von 280 kg/m³ und wird in Ölbinder mit 5 x 5 cm Kantenlänge aufgeteilt. Der Porenanteil der Binder liegt bei 80 Ma.-%, gemessen am Gesamtanteil des Ölbinders (gemessen mit Heliumpyknometer nach DIN 51913). Die Biegefestigkeit beträgt 1,98 N/mm² (nach DIN EN 310).

### Beispiel 1b: Herstellung des Ölbindematerials mit Naturlatex

Fichtenholzfaserstoff wurde in Wasser bei einer Temperatur von 50 °C eingeweicht (Feststoffgehalt 3 %). Im Anschluss wurden 7 % Naturlatex in einer Lösung zur Faserstoffsuspension gegeben während kontinuierlich gerührt wurde. Zur Fällung wurde Al₂(SO₄)₃ der Suspension zugesetzt. Danach wurde die Suspension wie in Beispiel 1a weiterverarbeitet. Die Rohdichte der Ölbinder beträgt 265 kg/m³ und der Porenanteil 81 Ma.-%, gemessen am Gesamtanteil des Ölbinders. Die Biegefestigkeit liegt bei 1,96 N/mm² (nach DIN EN 310).

### Beispiel 1c: Herstellung des Ölbindematerials mit Tannin

Fichtenholzfaserstoff wurde in Wasser bei einer Temperatur von 50 °C eingeweicht (Feststoffgehalt 3 %). Im Anschluss wurde 5 % Quebrachotannin zur Faserstoffsuspension gegeben, während kontinuierlich gerührt wurde. Zur Fällung wurde Al₂(SO₄)₃ der Suspension zugesetzt. Danach wurde die Suspension wie in Beispiel 1a und 1b weiterverarbeitet. Die Rohdichte der Ölbinder beträgt 279 kg/m³ und der Porenanteil 80 Ma.-%. Die Biegefestigkeit liegt bei 1,97 N/mm² (nach DIN EN 310).

### Beispiel 2: Einsatz des Materials als Ölbinder

Jeweils 50 g Rohöl wurden in Petrischalen gefüllt. Jeweils 11 g (40 cm³) des Ölbindematerials aus Beispiel 1a, 1b und 1c wurden dazugegeben. Nach 1 min hatte das Ölbindematerial jeweils ca. 27 g Öl (ca. 2,5 fache des Eigengewichtes) aufgenommen.

### Beispiel 3: Einsatz der Ölbinder im Öl-/Wassergemisch und Test der Langzeitschwimmfähigkeit

In einem Schüttler wurden in einen Behälter 1,5 I Wasser gefüllt. Dem Wasser werden 22,5 g Rohöl (0,3 mm Ölschichtdicke) und 11 g Ölbinder (aus Beispiel 1a) mit einem Volumen vom 40 cm³ zugegeben, was einem Bedeckungsgrad von 11 % entsprach. Der Behälter wurde mit einer Frequenz von 0,5 Hz bewegt. In den ersten 15 min wurde alle 5 min die Massezunahme gemessen, danach alle 10 min. Nach 10 min hatten die Binder eine Öl-/Wassermenge von 22 g aufgenommen, was einem Volumen von ca. 600 kg/m³ entspricht. Nach weiteren 80 min wurden insgesamt 25 g Öl-/Wassermenge aufgenommen. Eine Analyse des Restwassers im Behälter (Scheidetrichter) ergab, dass 18,41 g Öl und nur 7,31 g Wasser vom Binder aufgenommen wurden. Das entspricht einer Ölaufnahme von ca. 660 kg/m³ und einem Reinigungsgrad von ca. 85 %. Die Binder schwammen dann weitere 14 Tage im Schüttelwasserbad bevor der Versuch abgebrochen wurde.
Der Versuch wurde auch mit Ölbindern nach den Beispielen 1b und 1c durchgeführt, wobei die Ölaufnahme ähnlich wie bei den Ölbindern nach Beispiel 1a war und die Schwimmfähigkeit bei mindestens 8 Tagen lag.

## Patentansprüche

1. Poröser Ölbinder in Form eines Vlieses aus Fasern aus lignocellulosehaltigen Rohstoffen, **dadurch gekennzeichnet, dass**
a) die Dichte des Ölbinders 80 bis 300 kg/m³ beträgt,
b) der Ölbinder 3 bis 6 mm dick ist, wobei sich die Dicke auf die Länge der kürzesten Kante des Ölbinders bezieht,
c) die Breitfläche des Ölbinders eine Dimension von 9 bis 200 cm² hat,
d) der Porenanteil des Ölbinders, bestimmt nach DIN 51913, 30 bis 96 %, gemessen am Gesamtanteil des Ölbinders, beträgt und
e) die Biegefestigkeit des Ölbinders, bestimmt nach DIN EN 310, mindestens 1,5 N/mm² beträgt.

2. Ölbinder nach Anspruch 1, **dadurch gekennzeichnet, dass** der Ölbinder hydrophobierte Fasern aufweist.

3. Ölbinder nach einem der Ansprüche 1 oder 2, **dadurch gekennzeichnet, dass** die lignocellulosehaltigen Rohstoffe aus Holz, Getreide-, Flachs-, Raps-, Reis- oder Baumwollstroh, Kokosfasern, Bagasse, Bambus, Kork, Seegras, Baumrinde oder Mischungen aus diesen sind.

4. Ölbinder nach einem der Ansprüche 1 bis 3, **dadurch gekennzeichnet, dass** die lignocellulosehaltigen Rohstoffe thermisch modifiziert sind.

5. Ölbinder nach einem der Ansprüche 1 bis 4, **dadurch gekennzeichnet, dass** der Ölbinder tierische Rohstoffe mit einem Massenanteil von 5 und 15 Ma.-% aufweist.

6. Ölbinder nach einem der Ansprüche 2 bis 5, **dadurch gekennzeichnet, dass** die Fasern mit natürlichen oder naturidentischen Additiven ausgewählt aus Paraffinen, Wachsen, synthetischem oder natürlichem Latex, Rindenextrakten, Tanninen oder Mischungen aus diesen hydrophobiert sind.

7. Ölbinder nach einem der Ansprüche 1 bis 6, **dadurch gekennzeichnet, dass** die Faseroberfläche mit einem natürlichen oder synthetischen Bindemittel ausgewählt aus Stärke, Proteinen, Harnstoffharzen, Isocyanaten oder Mischungen aus diesen benetzt ist.

8. Ölbinder nach einem der Ansprüche 1 bis 7, **dadurch gekennzeichnet, dass** die Ausgleichsfeuchte des Ölbinders 5 bis 20 Ma.-%, gemessen an der atro Masse des Faserstoffes, beträgt.

9. Ölbinder nach einem der Ansprüche 1 bis 8, **dadurch gekennzeichnet, dass** die Oberfläche der Fasern oder des Ölbinders immobilisierte Mikroorganismen aufweist.

10. Ölbinder nach Anspruch 9, **dadurch gekennzeichnet, dass** die Mikroorganismengemeinschaft aus alkanotrophen Bakterien der Gattungen *Rhodococcus, Pseudomonas und Sphingomonas* sowie phototrophen Algen und Cyanobakterien der Gattungen *Microcoleus, Phormidium, Lyngbya, Oscillatoria und Anabaena* besteht.

11. Ölbinder nach einem der Ansprüche 9 bis 10, **dadurch gekennzeichnet, dass** die Mikroorganismen noch lyophilisierte oder in Suspension befindliche Mikroorganismen sind.

12. Verwendung eines Ölbinders nach einem der Ansprüche 1 bis 11 zum Abbau und/oder zur Entfernung von Ölverschmutzungen von der Wasseroberfläche in Meeren, Flüssen, Binnengewässern sowie Rückhaltebecken oder Abwasserbehandlungsanlagen.

## Claims

1. A porous oil binder in the form of a nonwoven material, consisting of fibres comprising lignocellulosic raw materials, **characterized in that**:
a) the density of the oil binder is 80 to 300 kg/m³,
b) the oil binder is 3 to 6 mm thick,
wherein the thickness refers to the length of the shortest edge of the oil binder
c) the large surface of the oil binder has a dimension of 9 to 200 cm²,
d) the pore fraction of the oil binder, determined according to DIN 51913, is 30 % to 96 %, measured over the entirety of the oil binder,
e) the bending strength of the oil binder, determined according to DIN EN 310, is at least 1.5 N/mm².

2. The oil binder according to claim 1, **characterized in that** the oil binder comprises hydrophobized fibres.

3. The oil binder according to claim 1 or claim 2, **characterized in that** the lignocellulosic raw material are obtained from wood, grain, flax, rape, rice or cotton straw, coconut fibres, bagasse, bamboo, cork, seaweed, tree bark or mixtures thereof.

4. The oil binder according to one of the claims 1 to 3, **characterized in that** the lignocellulosic raw materials are thermally modified.

5. The oil binder according to one of the claims 1 to 4, **characterized in that** the oil binder comprises animal-based raw materials in a proportion by weight in the range 5 % to 15 % by weight.

6. The oil binder according to one of the claims 2 to 5, **characterized in that** the fibres are hydrophobized with natural or nature-identical additives selected from paraffins, waxes, synthetic or natural latex, bark extracts, tannins or mixtures thereof.

7. The oil binder according to one of the claims 1 to 6, **characterized in that** the fibre surface is wetted with a natural or synthetic binder selected from starches, proteins, urea resins, isocyanates or mixtures thereof.

8. The oil binder as according to one of the claims 1 to 7, **characterized in that** the moisture content of the oil binder is 5 % to 20 % by weight, measured as the ATRO weight of the fibrous material.

9. The oil binder according to one of the claims 1 to 8, **characterized in that** the surface of the fibres or the oil binder comprises immobilized microorganisms.

10. The oil binder according to claim 9, **characterized in that** the microorganism community consists of alkanotrophic bacteria of the genuses *Rhodococcus, Pseudomonas* and *Sphingomonas* as well as phototrophic algae and cyanobacteria from the genuses *Microcoleus, Phormidium, Lyngbya, Oscillatoria* and *Anabaena.*

11. The oil binder according to one of the claims 1 to 10, **characterized in that** the microorganisms are still-lyophilised microorganisms or microorganisms in suspension.

12. Use of an oil binder according to one of the claims 1 to 11 for decomposition and/or removal of oil contamination on the water surface of seas, rivers, continental waters, retention basins and/or water treatment plants.

## Revendications

1. Absorbant d'huile poreux sous la forme d'un non-tissé en fibres de matières premières contenant de la lignocellulose, **caractérisé en ce que**
a) la densité de l'absorbant d'huile est de 80 à 300 kg/m³,
b) l'absorbant d'huile est d'une épaisseur de 3 à 6 mm, l'épaisseur se rapportant à la longueur de l'arête la plus courte de l'absorbant d'huile,
c) la face large de l'absorbant d'huile ayant une dimension de 9 à 200 cm²,
d) la proportion de pores de l'absorbant d'huile, déterminée selon DIN 51913 étant de 30 à 96 %, mesurée sur la part totale d'absorbant d'huile et
e) la résistance à la flexion de l'absorbant d'huile, déterminée selon DIN EN 310 étant d'au moins 1,5 N/mm².

2. Absorbant d'huile selon la revendication 1, **caractérisé en ce que** l'absorbant d'huile comporte des fibres rendues hydrophobes.

3. Absorbant d'huile selon l'une quelconque des revendications 1 ou 2, **caractérisé en ce que** les matières premières contenant de la lignocellulose sont du bois, de la paille, de la paille de lin, de la paille de colza, de la paille de riz ou de la bourre de coton, des fibres de coco, de la bagasse, du bambou, du liège, du crin végétal, de l'écorce ou des mélanges de ceux-ci.

4. Absorbant d'huile selon l'une quelconque des revendications 1 à 3, **caractérisé en ce que** les matières premières contenant de la lignocellulose sont thermiquement modifiées.

5. Absorbant d'huile selon l'une quelconque des revendications 1 à 4, **caractérisé en ce que** l'absorbant d'huile comporte des matières premières animales avec une part en masse de 5 et 15 % en masse.

6. Absorbant d'huile selon l'une quelconque des revendications 2 à 5, **caractérisé en ce que** les fibres sont rendues hydrophobes avec des additifs naturels ou identiques naturels choisis parmi les paraffines, les cires, le latex synthétique ou naturel, les extraits d'écorces, les tanins ou mélanges de ceux-ci.

7. Absorbant d'huile selon l'une quelconque des revendications 1 à 6, **caractérisé en ce que** la surface des fibres est mouillée d'un agent liant naturel ou synthétique, choisi parmi l'amidon, des protéines, des résines d'urée, des isocyanates ou des mélanges de ceux-ci.

8. Absorbant d'huile selon l'une quelconque des revendications 1 à 7, **caractérisé en ce que** l'humidité d'équilibre de l'absorbant d'huile est de 5 à 20 % en masse, mesurée sur la masse atro de la matière fibreuse.

9. Absorbant d'huile selon l'une quelconque des revendications 1 à 8, **caractérisé en ce que** la surface des fibres ou de l'absorbant d'huile comporte des microorganismes immobilisés.

10. Absorbant d'huile selon la revendication 9, **caractérisé en ce que** la communauté des microorganismes est composée de bactéries alcanotrophes des espèces *Rhodococcus, Pseudomonas* et *Sphingomonas,* ainsi que des algues phototrophes et des cyanobactéries des espèces *Microcoleus, Phormidium, Lyngbya, Oscillatoria* et Anabaena.

11. Absorbant d'huile selon l'une quelconque des revendications 9 à 10, **caractérisé en ce que** les microorganismes sont des microorganismes encore lyophilisés ou se trouvant en suspension.

12. Utilisation d'un absorbant d'huile selon l'une quelconque des revendications 1 à 11 pour dégrader et/ou pour éliminer des pollutions par hydrocarbures de la surface de l'eau dans des mers, des fleuves, des eaux intérieures, ainsi que dans des bassins de rétention ou des stations d'épuration.
